# EUROPEAN PATENT APPLICATION

(11) **EP 4 652 998 A1**
(43) Date of publication of application: **26.11.2025**
(21) Application number: 24177063.5
(22) Date of filing: 21.05.2024
(51) Int. Cl.: A61K 31/553, A61P 17/00, A61P 29/00

(54) **TREATMENT OF RECEPTOR-INTERACTING PROTEIN KINASE 1- MEDIATED DISEASE WITH ECLITASERTIB**

(71) Applicant: Technische Universität München, 80333 München (DE)
(72) Inventor: LAUFFER, Felix, München (DE); JARGOSCH, Manja, München (DE)
(74) Representative: Weickmann & Weickmann PartmbB

(57) **Abstract**

The present disclosure provides methods of treating receptor-interacting serine/threonine-protein kinase 1 (RIPK1) inhibitors with eclitasertib.

## Description

### FIELD OF THE DISCLOSURE

The present disclosure relates to the field of therapeutic kinase inhibitors, in particular receptor-interacting serine/threonine-protein kinase 1 (RIPK1) inhibitors, to treat receptor-interacting protein kinase 1-mediated disease.

### BACKGROUND

Chronic inflammatory skin diseases (ISD) are increasingly frequent in western countries and severely impact patient's quality of life. While there have been great achievements for the two most common ISD, psoriasis and atopic dermatitis, the majority of ISD lacks safe and effective treatments. Lichen planus (LP) and cutaneous lupus erythematosus (LE) are the two most prominent examples for type 1 mediated ISD. The estimated prevalence of LP is about 1%. LP has a wide clinical spectrum, classically presenting as itchy papules at the extremities and white net-shaped and erosive plaques at the oral and genital mucosa. However, there are also generalized, exanthematic LP subtypes as well as LP leading to nail dystrophy and scaring alopecia. LP is still treated with unspecific immunosuppressants, such as topical and systemic glucocorticosteroides, antimalarials, methotrexate or retinoids. However, these strategies often fail in reaching sufficient long term disease control or need to be discontinued due to side effects. So far, no targeted treatment for LP has been approved worldwide.

LP and LE are characterized by a lymphocytic infiltrate close to the epidermis, which causes cell death of basal keratinocytes. This immune reaction is named interface dermatitis (ID) and resembles a pattern of immune-epithelial interaction, which can also be found in Graft-versus-host disease, dermatomyositis, viral infections and as a side effect of immune check point inhibitors used for cancer treatment. LP has been found to be associated with genes belonging to type 1 immunity (*Tbx21, STAT1, IFNG, IRF1, IRF2, IRF3, IL12* or *IL12R*)*.* T cells isolated from LP has been shown to mainly produce IFN-γ and TNFα. In addition, it has been demonstrated that type 1 cytokines cause apoptosis and necroptosis of epidermal keratinocytes in LP.

IFN-γ and TNF-α initiate cell death by activating the Receptor-interacting serine/threonine-protein kinase 1 (RIPK1) signaling pathway. RIPK1 is an intracellular protein involved in the regulation of inflammation, cytokine release, and cell death. Upon activation of TNF receptor 1, RIPK1 is released from complex I and activated by autophosphorylation. Well-established phosphorylation at serine 166 serves as a biomarker for RIPK1 kinase-dependent pathologies. Activated RIPK1 can then form the two pro-death complexes IIa and IIb. The pro-apoptotic complex IIa, in which RIPK1, FADD and caspase 8 assemble, facilitates RIPK1-dependent apoptosis. When caspase-8 activity is impaired, necroptotic complex IIb is formed, which consists of RIPK1, FADD, caspase 8, RIPK3 and mixed lineage kinase domain like pseudokinase (MLKL). Here, activated RIPK1 promotes the activation and phosphorylation of RIPK3, which in turn activates and phosphorylates MLKL, finally resulting in the generation of pores in the outer cell membrane, as well as cell bursting and uncontrolled release of intracellular components - a process known as necroptosis. As a result, necroptosis is a strong inflammatory stimulus and accelerates local inflammation. Thus, targeting necroptosis might be a therapeutic option for treating Lichen planus.

Eclitasertib (also known as SAR443122 or DNL758) is an inhibitor of RIPK1 and represents a novel therapeutic approach for the treatment of Lichen planus.

### BRIEF DESCRIPTION

In one embodiment, the present disclosure provides a method of treating lichen planus, comprising administering to a subject in need thereof a therapeutically effective amount of a RIPK1 inhibitor (RIPK1i).

In one embodiment, the present disclosure provides a method of treating cutaneous lupus erythematosus in a subject in need thereof, comprising administering to the subject in need thereof a therapeutically effective amount of a RIPK1 inhibitor (RIPK1i), wherein the therapeutically effective amount of the RIPK1i is sufficient to reduce the level of one or more of IFNg, TNF, CXCL9/10/11, CCL3 and IL1RA.

In one embodiment, the present disclosure provides a method of normalizing or improving skin architecture in a subject in need thereof, comprising administering to the subject in need thereof a therapeutically effective amount of a RIPK1i.

In one embodiment, the present disclosure provides a method of normalizing or restoring normal epidermal in a subject in need thereof, comprising administering to the subject in need thereof a therapeutically effective amount of a RIPK1i.

In one embodiment, the present disclosure provides method of reducing epidermal thickness in a subject in need thereof, comprising administering to the subject in need thereof a therapeutically effective amount of a RIPK1i.

### BRIEF DESCRIPTION OF FIGURES

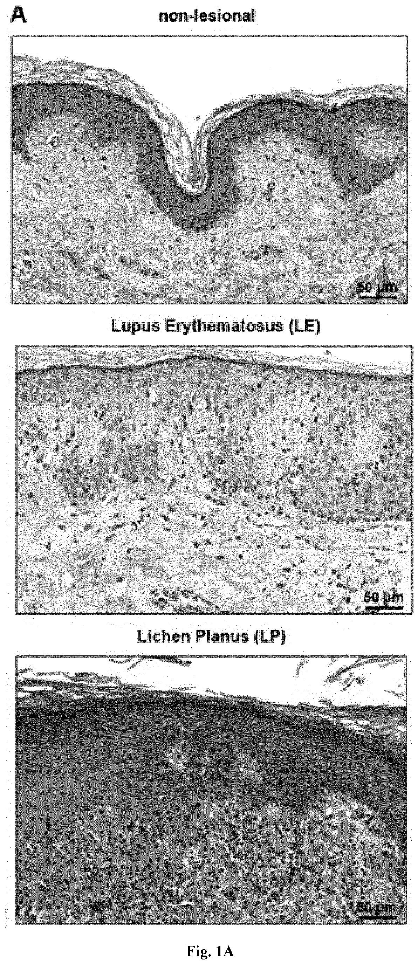

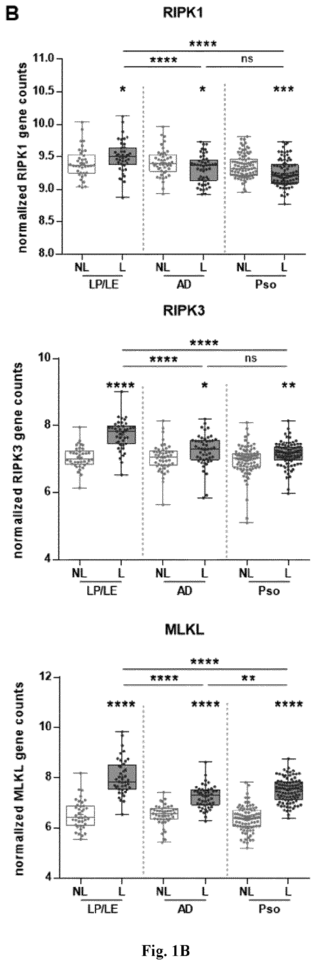

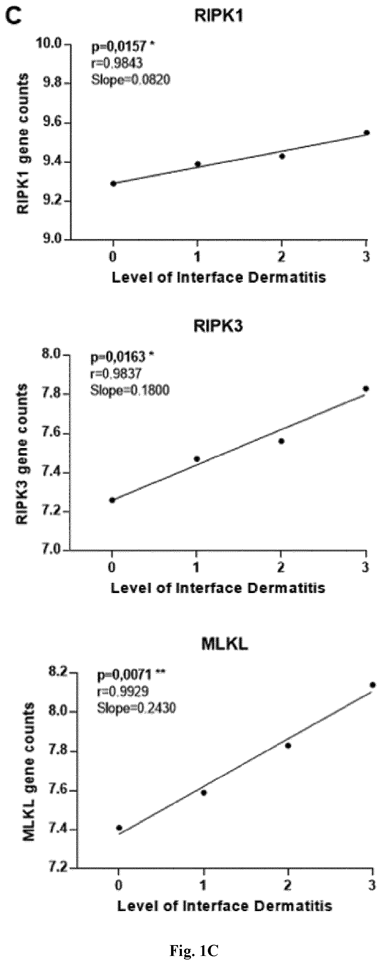

Fig. 1: RIPK1, RIPK3 and MLKL are significantly upregulated in lesional skin of LP and LE patients and correlates positively with interface dermatitis. A) Representative histological HE staining of non-lesional and lesional skin from a lupus erythematosus (LE) and lichen planus (LP) patient. Scale bar indicates 50 µm. B) Normalized gene counts of RIPK1, RIPK3 and MLKL in non-lesional (NL) and lesional (L) skin of LP/LE (n=41), AD (n=48) and psoriasis (n=90) patients. Differences between lesional and non-lesional group was analyzed with an unpaired t test with Welch's correction. Comparison of patients groups was performed using 1way ANOVA test (Uncorrected Fisher's LSD). C) Correlation of normalized gene counts for RIPK1, RIPK3 or MLKL to the level of Interface Dermatitis in ISD (n=274) with 0 (no ID), 1 (mild ID), 2 (moderate ID) and 3 (severe ID). Significance of linear regression was calculated with Pearson correlation. *P<0.05, **P<0.01, ***P<0.001, ****P<0.0001.

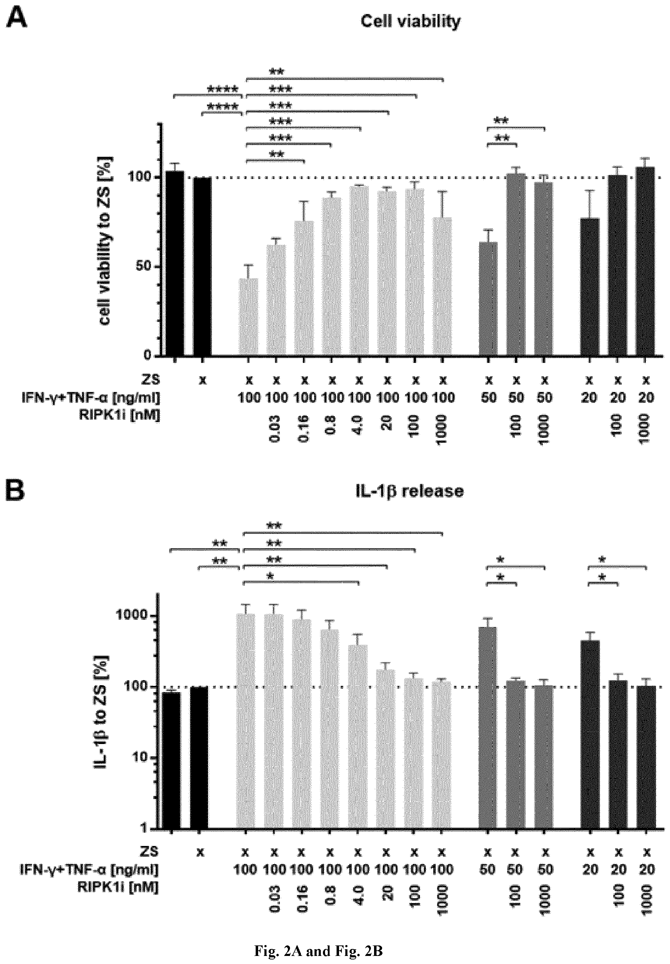

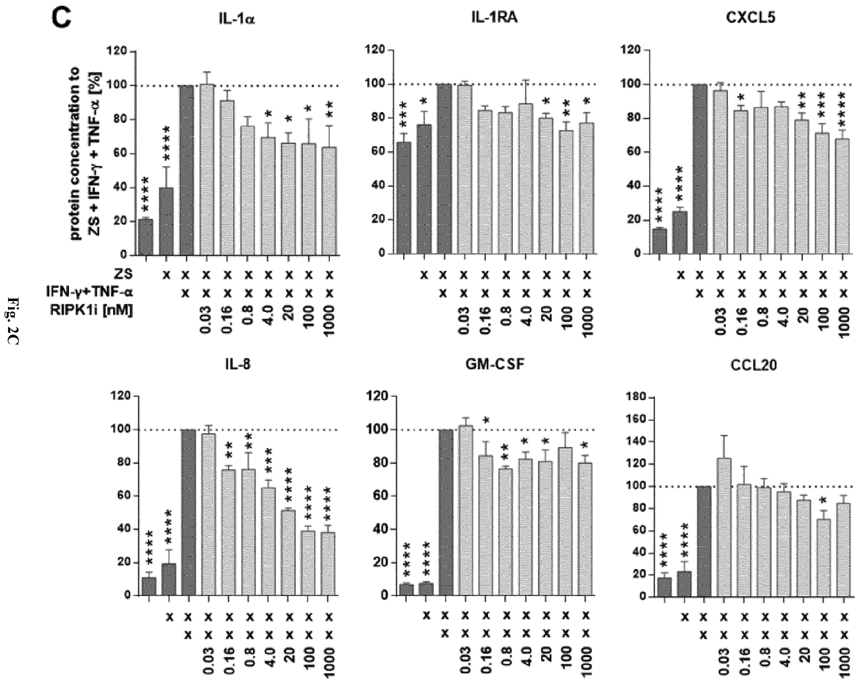

Fig. 2: Inhibition of RIPK1 in primary human keratinocytes diminishes cell death induction, inflammasome activation and inflammation. A-C) Cell viability (A, n=4), activity of inflammasome measured by the IL-1β release (B, n=4) and protein concentrations of secreted inflammatory cytokines (C, n=3, 100 ng/ml IFN-γ + TNF-α) of primary human keratinocytes 24 h after cell death induction with IFN-γ + TNF-α and zVAD [20 µM] + SMAC [1 µM] (ZS) and treatment with indicated concentrations of RIPK1 inhibitor. Comparison of treatment groups was performed using 1way ANOVA test (Uncorrected Fisher's LSD).

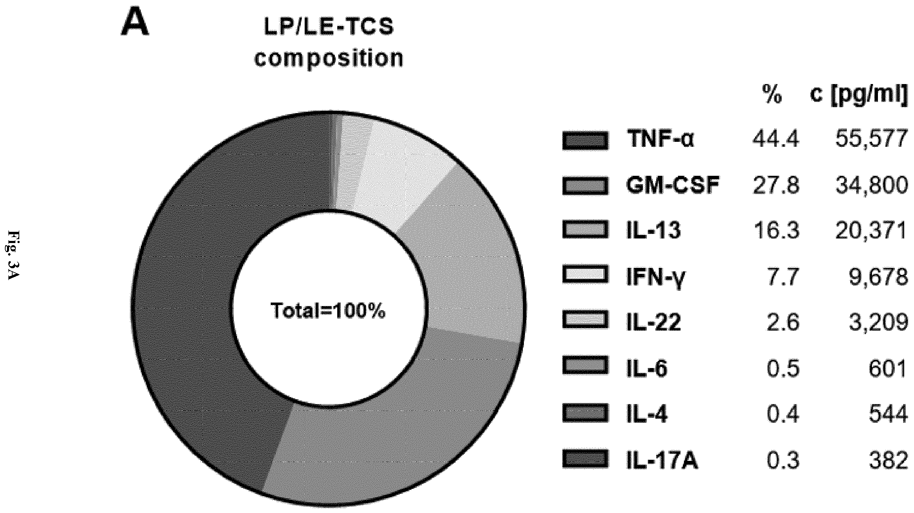

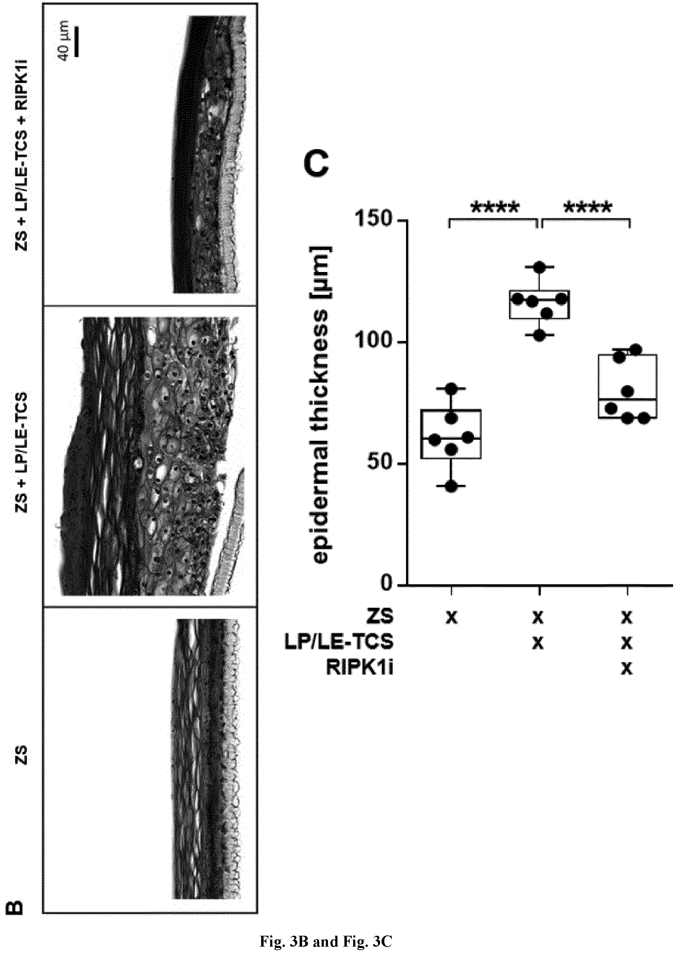

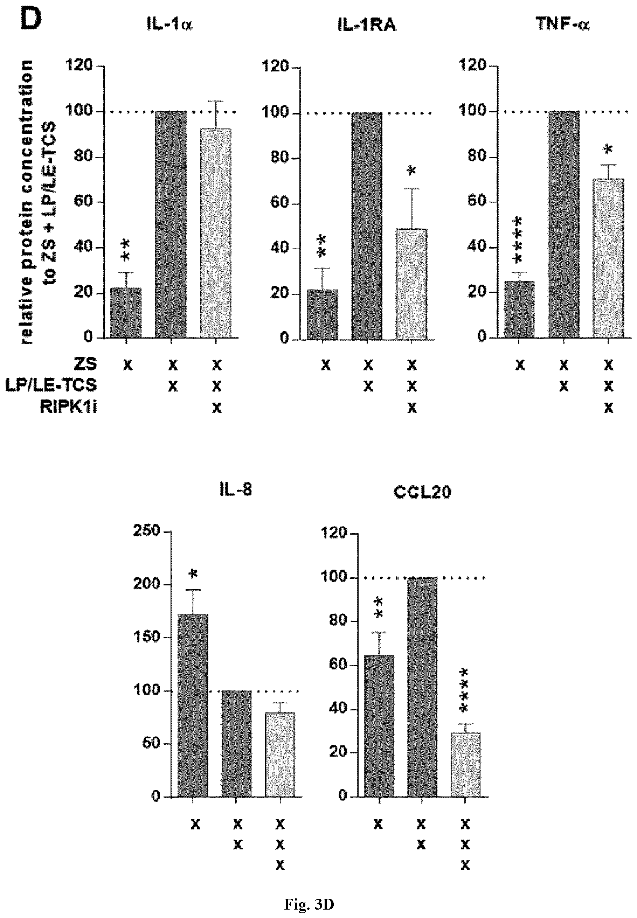

Fig. 3: Inhibition of RIPK1 in human keratinocyte skin equivalents significantly reduces cell death and inflammation under necroptotic conditions. A) LP/LE-TCS composition analyzed by Bio-Plex technique. Lesional T cell supernatant mix of 5 LP and 3 LE donors. T cell specific cytokines were visualized as percentages of 100 % in total and as absolute concentrations in pg/ml. B) Representative H&E staining of keratinocyte skin equivalents (n=3) 72 h after induction of cell death with LP/LE-TCS [1:10 diluted] and treatment with RIPK1 inhibitor (100 nM) under necroptotic conditions with zVAD [20 µM] and SMAC [1 µM] (ZS). Scale bar indicates 40 µm. C) Quantification of the epidermal thickness (w/o stratum cornium) of the keratinocyte skin equivalents in (B) under necroptotic conditions (n=3 donors in duplicates). D) Relative concentration of secreted cytokines in the SN of keratinocyte skin equivalent cultures in (B) to the stimulated control sample (ZS + LP/LE-TCS) without RIPK1 inhibitor treatment (n=3). Comparison of treatment groups was performed using 1way ANOVA test (Uncorrected Fisher's LSD).

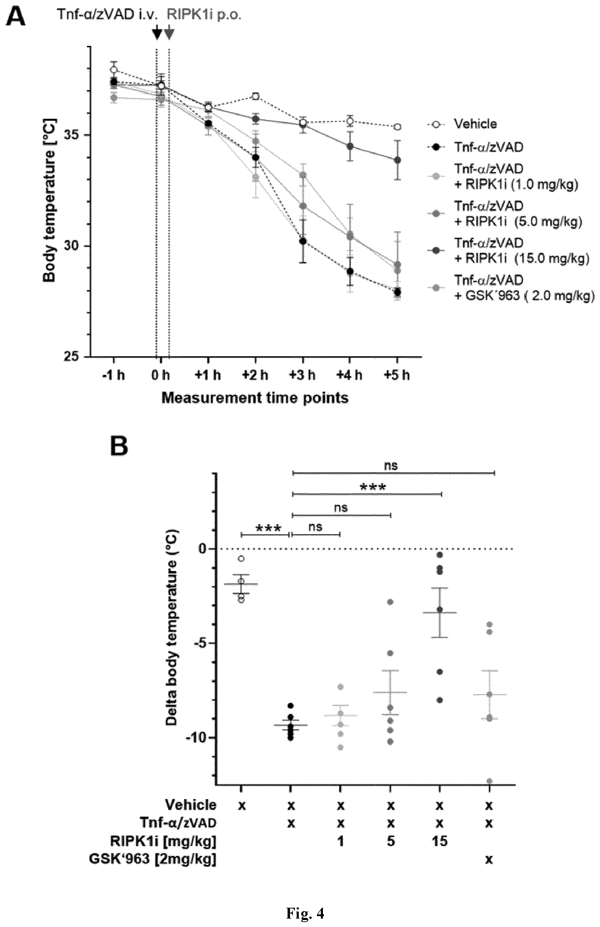

Fig. 4: RIPK1 inhibitors specifically block TNF-α-induced SIRS in vivo. In vivo testing of RIPK1 inhibitor by inhibition of hypothermia in a TNF-α induced systemic inflammatory response syndrome (SIRS) model in mice. C57BL/6 mice (n=6 per group) received vehicle or murine TNF-α (0.3 mg/kg) and zVAD (16.7 mg/kg) dissolved in vehicle by i.v. injection to induce a systemic inflammatory response (timepoint = 0 h). RIPK1 inhibitor SAR443122 (1, 5 and 15 mg/kg) or GSK'963 (2 mg/kg) were dosed orally 15 min after induction of hypothermia. Body temperature was recorded every hour up to 5 h and visualized in (A). Drop of body temperature at 5 h timepoint after induction of hypothermia was calculated in (B). Comparison of treatment groups was performed with 1way ANOVA test with Dunnett's multiple correction.

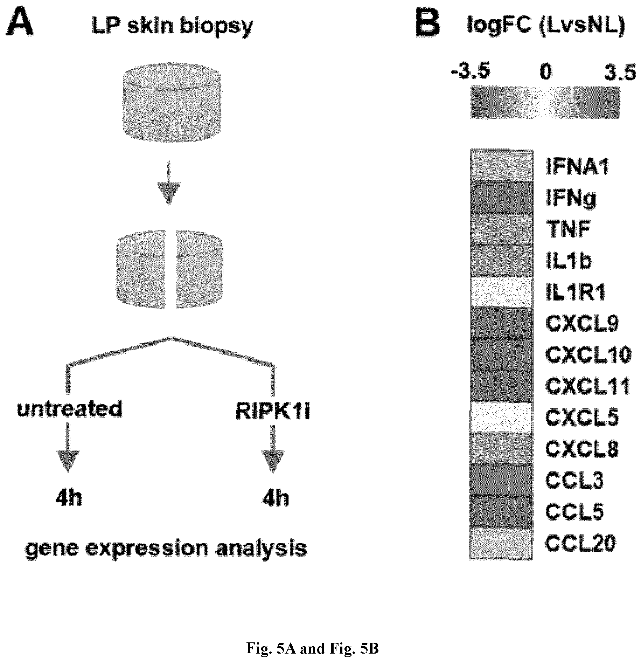

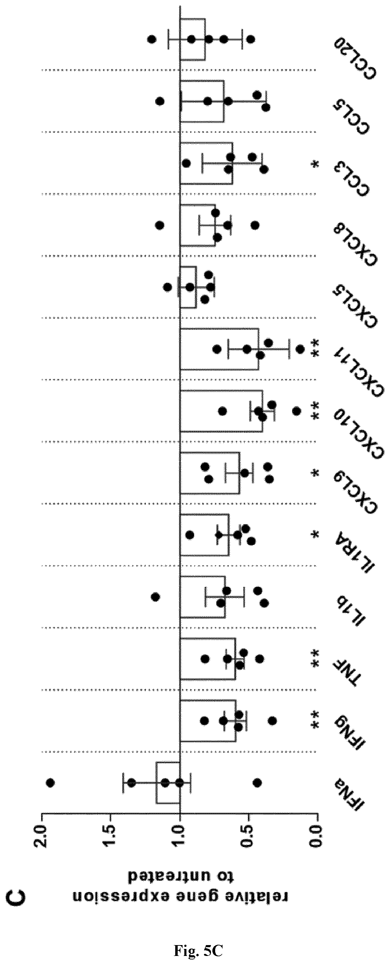

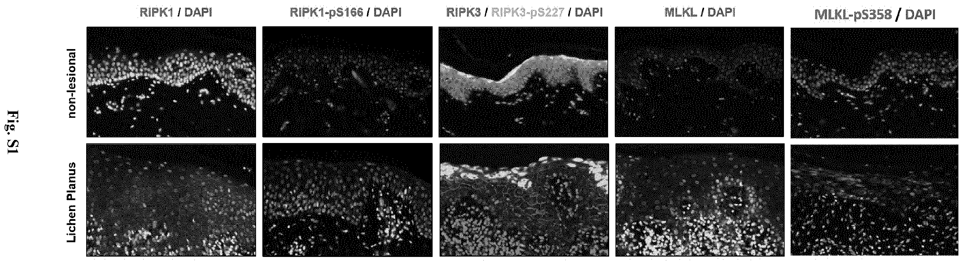

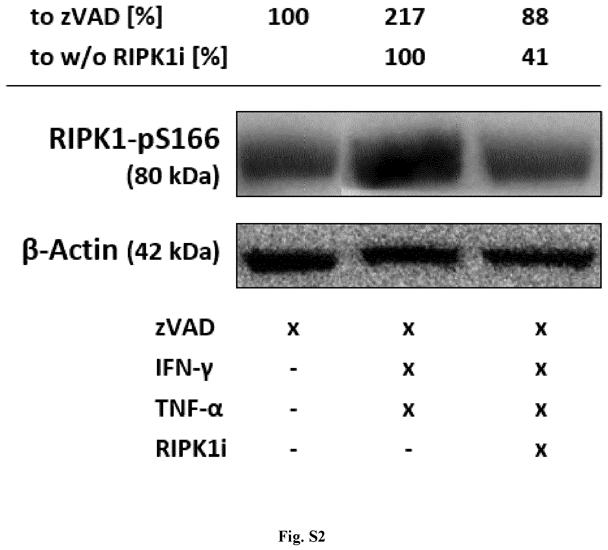

Fig. 5: RIPK1 inhibition in cultured lesional skin from Lichen Planus patients suppresses the expression of selected disease-related genes. A) Experimental setup: 6 mm punch biopsies of lesional skin were collected, divided in two parts, and incubated for 4 h with either vehicle or RIPK1 inhibitor (1 µM), followed by gene expression quantification. B) LogFC of selected differentially expressed genes in human lesional skin biopsies of LP patients (n=30) compared to autologous non-lesional skin. C) Gene expression of selected genes in human lesional skin biopsies of LP patients (n=5) after RIPK1 inhibitor treatment relative to vehicle-treated samples. Comparison of disease groups was performed using unpaired t test with Welch's correction.
Fig. S1: Representative immuno-fluorescence staining of (p)RIPK1, (p)RIPK3 and (p)MLKL in non-lesional and lesional skin of Lichen planus patients. Sections were counterstained with DAPI (blue).
Fig. S2: RIPK1 inhibitor efficiently blocks S166 autophosphorylation of RIPK1. Detection of RIPK1-pS 166 in WB analysis of primary human keratinocytes 6 h after cell death induction with IFN-γ [10 ng/ml] + TNF-α [10 ng/ml] + zVAD [20 µM] and treatment with

RIPK1 inhibitor [100 nM]. Relative protein concentrations are calculated to zVAD and to zVAD + IFN-γ + TNF-α without RIPK1i and normalized to β-Actin.

### DETAILED DESCRIPTION

While the disclosure provides illustrated embodiments, it will be understood that they are not intended to limit the disclosure to those embodiments. On the contrary, the disclosure is intended to cover all alternatives, modifications, and equivalents, which may be included within the disclosure as defined by the appended claims.

Unless otherwise stated, the following terms used in the specification and claims are defined for the purposes of this disclosure and have the following meaning:
The phrase "pharmaceutically acceptable" is employed herein to refer to those compounds, materials, compositions and/or dosage forms which are, within the scope of sound medical judgment, suitable for use in contact with the tissues of human beings without excessive toxicity, irritation, allergic response or other problem or complication, commensurate with a reasonable benefit/risk ratio.

As used herein, the term "pharmaceutically acceptable salt" refers to the relatively non-toxic, inorganic and organic acid addition salts, and base addition salts, of a compound. These salts can be prepared in situ during the final isolation and purification of the compounds.

A "pharmaceutically acceptable carrier" or a "pharmaceutically acceptable excipient" means a carrier or an excipient that is useful in preparing a pharmaceutical composition that is generally safe, non-toxic and neither biologically nor otherwise undesirable, and includes a carrier or an excipient that is acceptable for veterinary use as well as human pharmaceutical use. "A pharmaceutically acceptable carrier/excipient" as used in the specification and claims includes both one and more than one such excipient.

"Treating" or "treatment" of a disease includes: 1) inhibiting the disease, *e*.*g*., arresting or reducing the development of the disease or its clinical symptoms; inhibiting further progression or worsening of at least one symptom, i.e. by reducing the severity or frequency of at least one symptom; 2)relieving the disease, *e*.*g*., causing regression of the disease or its clinical symptoms.

"Subject" refers to a human or animal. In some embodiments, the subject is human (i.e., a "patient"). In some embodiments, the subject is an animal.

A "therapeutically effective amount" means the amount of the RIPK1 inhibitor compound, that, when administered to a mammal for treating a disease, is sufficient to effect such treatment for the disease. The "therapeutically effective amount" will vary depending on the compound, the disease and its severity and the age, weight, etc., of the mammal to be treated.

Before describing the present teachings in detail, it is to be understood that the disclosure is not limited to specific compositions or process steps, as such may vary.

It should be noted that, as used in this specification and the appended claims, the singular form "a", "an" and "the" include plural references unless the context clearly dictates otherwise. Thus, for example, reference to "a conjugate" includes a plurality of conjugates and reference to "a cell" includes a plurality of cells and the like.

Numeric ranges are inclusive of the numbers defining the range. Measured and measurable values are understood to be approximate, taking into account significant digits and the error associated with the measurement. Also, the use of "comprise", "comprises", "comprising", "contain", "contains", "containing", "include", "includes", and "including" are not intended to be limiting. It is to be understood that both the foregoing general description and detailed description are exemplary and explanatory only and are not restrictive of the teachings.

Unless specifically noted in the above specification, embodiments in the specification that recite "comprising" various components are also contemplated as "consisting of" or "consisting essentially of" the recited components; embodiments in the specification that recite "consisting of" various components are also contemplated as "comprising" or "consisting essentially of" the recited components; and embodiments in the specification that recite "consisting essentially of" various components are also contemplated as "consisting of" or "comprising" the recited components (this interchangeability does not apply to the use of these terms in the claims.)

Unless specifically noted in the above specification, embodiments in the specification that recite "dose" weight described herein refers to the weight of free base of a compound (active moiety).

"Or" is used in the inclusive sense, *i.e.,* equivalent to "and/or," unless the context requires otherwise.

### ABBREVIATIONS

AD: Atopic Dermatitis
BCA: bicinchoninic acid
CCL: chemokine ligand
CXCL: C-X-C motif chemokine ligand
DAPI: 4',6-diamidino-2-phenylindole
DMSO: dimethyl sulfoxide
*FADD: Fas-associated death domain*
FC: fold change
GM-CSF: granulocyte macrophage colony-stimulating factor
ID: interface dermatitis
*IFNG:* interferon gamma
IFN-γ: interferon gamma
*IRF,* interferon regulatory factor
*IL: interleukin*
ISD: inflammatory skin disease
i.v.: intravenous
L: lesional
LE: lupus erythematosus
LP: lichen planus
MLKL: mixed lineage kinase domain like pseudokinase
MTS: 3-(4,5-dimethylthiazol-2-yl)-5-(3-carboxymethoxyphenyl)-2-(4-sulfophenyl)-2H-tetrazolium
NL: non-lesional
ns: non-significant
p.o.: peroral
PEG 200: Poly(ethylene glycol),Polyglycol
PMS: phenazine methyl sulphate
PMSF: protease Inhibitor Cocktail Set III, Animal-Free,
RIPA: radio immunoprecipitation assay
RIPK: receptor-interacting serine/threonine-protein kinase
RIPK1i: RIPK1 inhibitor
S: SMAC
SIRS: systemic immune response syndrome
SLE: systemic lupus erythematosus
SN: supernatant
SDS-PAGE: sodium dodecyl-sulfate polyacrylamide gel electrophoresis
SMAC: second mitochondria-derived activator of caspase
*STAT1:* signal transducer and activator of transcription 1
*Tbx21*: T-box transcription factor
*TNF*: tumor necrosis factor
Th: T helper cell
US: unstimulated
zVAD (Z): z-Val-Ala-DL-Asp(Ome)-fluoromethylketone

### Eclitasertib :

Eclitasertib is a novel, potent and selective Receptor-Interacting serine-threonine Protein Kinase1 (RIPK1) inhibitor. Eclitasertib has the chemical name 5-Benzyl-N-[(3S)-5-methyl-4-oxo-2,3-dihydropyrido[3,2-b][1,4]oxazepin-3-yl]-4H-1,2,4-triazole-3-carboxamide (also known as (S)-5-benzyl-N-(5-methyl-4-oxo-2,3,4,5-tetrahydropyrido[3,2-b][1,4]oxazepin-3-yl)-4H-1,2,4-triazole-3-carboxamide). It is described in the patent U.S. Patent No. 9,896,458. Eclitasertib has the following structural formula (I) (also referred as Compound (1), SAR443122):

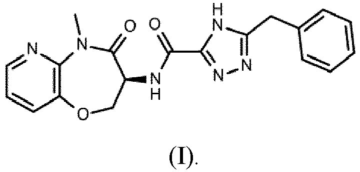

As used herein, eclitasertib may be provided in a pharmaceutical composition comprising a therapeutically effective amount of eclitasertib or a pharmaceutically acceptable salt thereof. The pharmaceutical composition may typically be in the form of, e.g., a liquid solution, dispersion, suspension, tablet, capsule, or the like. The pharmaceutical composition may comprise inactive ingredients that are pharmaceutically acceptable excipients and/or carriers. Eclitasertib is typically administered orally (i.e., p.o., PO, or per os).

### Treatment

### Non-limiting embodiments of the disclosure:

In one embodiment, herein disclosed is a method of treating lichen planus (LP) in a subject in need thereof, comprising administering to the subject in need thereof a therapeutically effective amount of a RIPK1 inhibitor (RIPK1i).

In one embodiment, herein disclosed is method of treating lichen planus (LP) in a subject in need thereof, comprising administering to the subject in need thereof a therapeutically effective amount of Compound (I) or pharmaceutically acceptable salt thereof.

In one embodiment, herein disclosed is use of eclitasertib for preparing a medicament for the treatment of lichen planus.

In one embodiment, herein disclosed is Eclitasertib for use in treating lichen planus.

In one embodiment, herein disclosed is use of eclitasertib for preparing a medicament for the treatment of lichen planus.

In one embodiment, herein disclosed is a method of treating lichen planus (LP) in a subject in need thereof, comprising administering to the subject in need thereof a therapeutically effective amount of a RIPK1 inhibitor (RIPK1i), wherein the therapeutically effective amount of the RIPK1i is sufficient to reduce the level of one or more of *IFNg, TNF, CXCL9*/*10*/*11, CCL3* and *IL1RA.*

In one embodiment, herein disclosed is a method of treating lichen planus (LP) in a subject in need thereof, comprising administering to the subject in need thereof a therapeutically effective amount of Compound (I) or pharmaceutically acceptable salt thereof, wherein the therapeutically effective amount of the RIPK1i is sufficient to reduce the level of one or more of *IFNg, TNF, CXCL9*/*10*/*11, CCL3* and *IL1RA.*

In one embodiment, herein disclosed is a method of treating cutaneous lupus erythematosus (LE) in a subject in need thereof, comprising administering to the subject in need thereof a therapeutically effective amount of a RIPK1 inhibitor (RIPK1i), wherein the therapeutically effective amount of the RIPK1i is sufficient to reduce the level of one or more of *IFNG, TNF, CXCL9*/*10*/*11, CCL3* and *IL1RA.*

In one embodiment, herein disclosed is a method of treating cutaneous lupus erythematosus (LE) in a subject in need thereof, comprising administering to the subject in need thereof a therapeutically effective amount of Compound (I) or pharmaceutically acceptable salt thereof, wherein the therapeutically effective amount of the RIPK1i is sufficient to reduce the level of one or more of *IFNg*, *TNF, CXCL9*/*10*/*11, CCL3* and *IL1RA.*

In one embodiment, herein disclosed is a method of treating or reducing LP-related inflammation in a subject in need thereof, comprising administering to the subject in need thereof a therapeutically effective amount of a RIPK1i.

In one embodiment, herein disclosed is a method of treating reducing LP-related inflammation in a subject in need thereof, comprising administering to the subject in need thereof a therapeutically effective amount of Compound (I) or pharmaceutically acceptable salt thereof.

In one embodiment, herein disclosed is use of eclitasertib for preparing a medicament for the treatment or reducing of LP-related inflammation.

In one embodiment, herein disclosed is eclitasertib for use in treating or reducing LP-related inflammation.

In one embodiment, herein disclosed is use of eclitasertib for preparing a medicament for the treatment or reducing of LP-related inflammation.

A method of treating or reducing LE-related inflammation in a subject in need thereof, comprising administering to the subject in need thereof a therapeutically effective amount of a RIPK1i.

In one embodiment, herein disclosed is a method of treating or reducing LE-related inflammation in a subject in need thereof, comprising administering to the subject in need thereof a therapeutically effective amount of Compound (I) or pharmaceutically acceptable salt thereof.

In one embodiment, herein disclosed is use of eclitasertib for preparing a medicament for the treatment or reduction of LE-related inflammation.

In one embodiment, herein disclosed is eclitasertib for use in treating or reducing LE-related inflammation.

In one embodiment, herein disclosed is use of eclitasertib for preparing a medicament for the treatment or reduction of LE-related inflammation.

In one embodiment, herein disclosed is method of treating or reducing LP-related hypothermia in a subject in need thereof, comprising administering to the subject in need thereof a therapeutically effective amount of a RIPK1i.

In one embodiment, herein disclosed is method of treating or reducing LP-related hypothermia in a subject in need thereof, comprising administering to the subject in need thereof a therapeutically effective amount of Compound (I) or pharmaceutically acceptable salt thereof. Use of eclitasertib for preparing a medicament for the treatment or reduction of LP-related hypothermia.

In one embodiment, herein disclosed is eclitasertib for use in treating or reducing LP-related hypothermia.

In one embodiment, herein disclosed is use of eclitasertib for preparing a medicament for the treatment or reduction of LP-related hypothermia.

In one embodiment, herein disclosed is a method of treating LE-related hypothermia in a subject in need thereof, comprising administering to the subject in need thereof a therapeutically effective amount of a RIPK1i.

In one embodiment, herein disclosed is a method of treating LE-related hypothermia in a subject in need thereof, comprising administering to the subject in need thereof a therapeutically effective amount of Compound (I) or pharmaceutically acceptable salt thereof.

In one embodiment, herein disclosed is use of eclitasertib for preparing a medicament for the treatment of LE-related hypothermia.

In one embodiment, herein disclosed is eclitasertib for use in treating LE-related hypothermia.

In one embodiment, herein disclosed is use of eclitasertib for preparing a medicament for the treatment of LE-related hypothermia.

In one embodiment, herein disclosed is a method of normalizing or improving LP-related skin architecture in a subject in need thereof, comprising administering to the subject in need thereof a therapeutically effective amount of a RIPK1i.

In one embodiment, herein disclosed is a method of normalizing or improving LP-related skin architecture in a subject in need thereof, comprising administering to the subject in need thereof a therapeutically effective amount of Compound (I) or pharmaceutically acceptable salt thereof.

In one embodiment, herein disclosed is use of eclitasertib for preparing a medicament for normalization or improvement of LP-related skin architecture.

In one embodiment, herein disclosed is eclitasertib for use in normalizing or improving LP-related skin architecture.

In one embodiment, herein disclosed is use of eclitasertib for preparing a medicament for normalization or improvement of LP-related skin architecture.

In one embodiment, herein disclosed is a method of normalizing or improving LE-related skin architecture in a subject in need thereof, comprising administering to the subject in need thereof a therapeutically effective amount of a RIPK1i.

In one embodiment, herein disclosed is a method of normalizing or improving LE-related skin architecture in a subject in need thereof, comprising administering to the subject in need thereof a therapeutically effective amount of Compound (I) or pharmaceutically acceptable salt thereof.

In one embodiment, herein disclosed is use of eclitasertib for preparing a medicament for normalization or improvement of LE-related skin architecture.

In one embodiment, herein disclosed is eclitasertib for use in normalizing or improving LE-related skin architecture.

In one embodiment, herein disclosed is use of eclitasertib for preparing a medicament for normalization or improvement of LE-related skin architecture.

In one embodiment, herein disclosed is a method of normalizing or improving skin architecture in a subject in need thereof, comprising administering to the subject in need thereof a therapeutically effective amount of a RIPK1i.

In one embodiment, herein disclosed is a method of normalizing or improving skin architecture in a subject in need thereof, comprising administering to the subject in need thereof a therapeutically effective amount of Compound (I) or pharmaceutically acceptable salt thereof.

In one embodiment, herein disclosed is use of eclitasertib for preparing a medicament for normalization or improvement of skin architecture.

In one embodiment, herein disclosed is eclitasertib for use in normalizing or improving skin architecture.

In one embodiment, herein disclosed is use of eclitasertib for preparing a medicament for normalization or improvement of skin architecture.

In one embodiment, herein disclosed is a method of normalizing or restoring normal epidermal in a subject in need thereof, comprising administering to the subject in need thereof a therapeutically effective amount of a RIPK1i.

In one embodiment, herein disclosed is a method of normalizing or restoring normal epidermal in a subject in need thereof, comprising administering to the subject in need thereof a therapeutically effective amount of Compound (I) or pharmaceutically acceptable salt thereof.

In one embodiment, herein disclosed is use of eclitasertib for preparing a medicament for normalization or restoration of normal epidermal

In one embodiment, herein disclosed is eclitasertib for use in normalizing or restoring normal epidermal.

In one embodiment, herein disclosed is use of eclitasertib for preparing a medicament for normalization or restoration of normal epidermal.

In one embodiment, herein disclosed is a method of reducing epidermal thickness in a subject in need thereof, comprising administering to the subject in need thereof a therapeutically effective amount of a RIPK1i.

In one embodiment, herein disclosed is a method of reducing epidermal thickness in a subject in need thereof, comprising administering to the subject in need thereof a therapeutically effective amount of Compound (I) or pharmaceutically acceptable salt thereof.

In one embodiment, herein disclosed is use of eclitasertib for preparing a medicament for reduction of epidermal thickness.

In one embodiment, herein disclosed is eclitasertib for use in reducing epidermal thickness.

In one embodiment, herein disclosed is use of eclitasertib for preparing a medicament for reduction of epidermal thickness.

### Materials and Methods

### Study cohort

Human skin samples were obtained from the Biobank Biederstein. 6 mm punch biopsies of lesional and non-lesional skin were collected under local anesthesia. Biopsies were divided into three parts: one part was fixed in 4 % formalin for histology, one part was collected in RNALater^{®} stabilization solution (Qiagen) at -80 °C until RNA preparation and one part was directly used for isolation of lesional T cells. For human lesional skin biopsy cultures separated 6 mm punch biopsies were used. Summary of patients characteristics are listed in Table S 1 for the bulk RNAseq Cohorte of LP/LE (n=41), AD (n=48) and psoriasis (n=90) skin lesions and in Table S2 for the RIPK1 inhibitor treatment of human lesional skin biopsies of LP patients (n=5).

C57BL/6 mice (male, with a weight of 20-27 g) were obtained from Charles River, Munich. Animal experiments were approved by the government of Hessia (FH-1036).

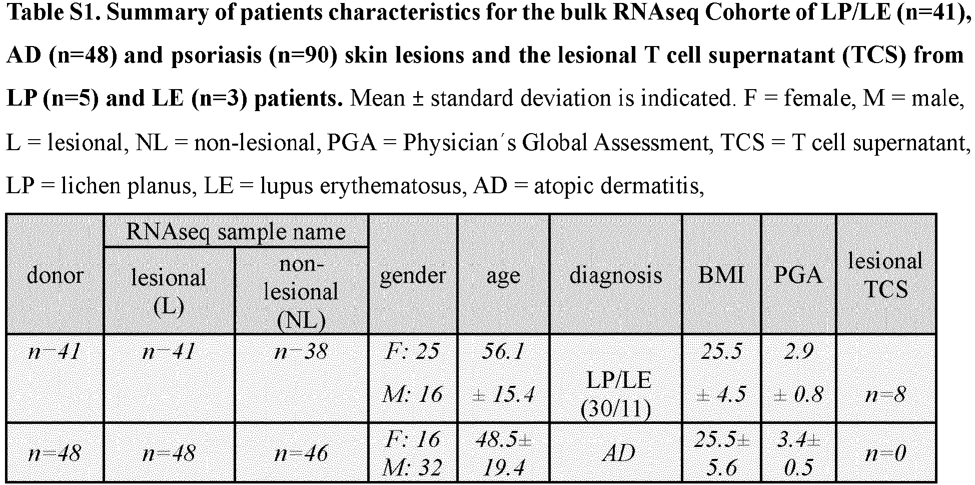

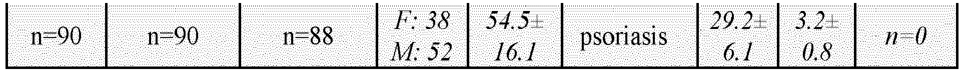

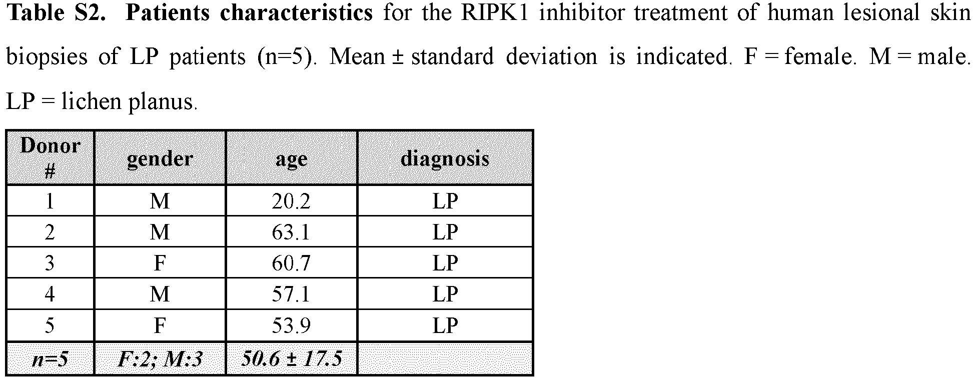

### Bulk RNAseq analysis of skin biopsies

Gene expression data for LP/LE (n=41), AD (n=48) and Pso (n=90) was derived from a study cohort published before (bioRxiv, 2020.2007.2025.221309 (2020). RNAseq libraries were generated using the TruSeq Stranded Total RNA Kit (Illumina) according to manufacturer's high sample protocol. Samples were sequenced on an Illumina HiSeq4000 as paired-end with a read length of 2x 150 bp and an average output of 40 Mio reads per sample and end. Sequence alignment was performed using STAR aligner with human genome reference hg38. RNAseq count data were normalized and then transformed using variance stabilizing transformation (VST) from the Bioconductor package DESeq2 (normalized gene counts).

### RIPK1 inhibitor

RIPK1 inhibitor SAR443122 also known as DNL758 (Denali Therapeutics) was provided by SANOFI. SAR443122 is currently under investigation in several clinical trials (ClinicalTrials.gov Identifier: NCT04469621, NCT05588843, NCT04781816). RIPK1 inhibitor is solved in DMSO to provide a 10 mM stock solution. For animal experiments RIPK1 inhibitors were dosed in 0.5 % Methylcellulose (MC), 0.1 % Tween80. Used concentrations are indicated in each section.

### Culture and cell death induction of primary human keratinocytes

Primary human keratinocytes were obtained from healthy donors by suction blister as reported before (J Allergy Clin Immunol 141, 1320-1333 e1311 (2018*)*) and donor characteristics are listed in Table S3. Epidermal keratinocytes were cultured in keratinocyte medium (DermaLife Basal Medium supplemented with DermaLife K LifeFactor Kit (Lifeline Cell Technology, LL-0007)) at 37 °C, 5 % CO₂ and second- to third-passages were used. At 70-80 % of confluence, keratinocytes were pre-incubated for 1 h with RIPK1 inhibitor (100 nM or as indicated, SANOFI) followed by stimulation with rhIFN-γ (100 ng/ml or as indicated, R&D, #285-IF-100/CF) and rhTNF-α (100 ng/ml or as indicated, R&D, #210_TA/CF) in the presence of zVAD (20 µM, Cell Signaling, #60332), SMAC (1 µM, LC Laboratories, #B-9135) and the RIPK1 inhibitor for 24 h in keratinocyte medium without hydrocortisone.

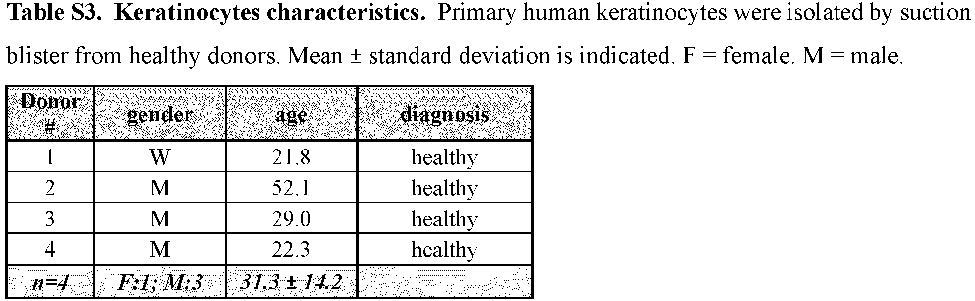

### Three-dimensional skin equivalents

Three-dimensional keratinocyte skin equivalents were generated as described before Arch Dermatol Res 296, 203-211 (2004)). Briefly, 3.0E⁵ primary human keratinocytes were seeded in 500 µL keratinocyte medium with 1.5 mM CaCh into the insert and placed in a well containing the same medium. After two days, keratinocytes were exposed to the air-liquid interface and medium in the surrounding well was supplemented with 50 µg/mL Vitamin C. Nine days after airlift, the skin equivalents were pre-treated for 1 h with RIPK1 inhibitor (100 nM, SANOFI) followed by a stimulation with supernatant mix of lesional T cells from LP and LE patients (1: 10 diluted) in the presence of zVAD (20 µM, Cell Signaling, #60332), SMAC (1 µM, LC Laboratories, #B-9135) and the RIPK1 inhibitor for 72 h. Afterwards, membranes of the fixated skin equivalents were cut out with a scalpel, divided into two pieces, formalin fixed and embedded in paraffin.

### Isolation of lesional T cells from LP and LE skin biopsies and production of supernatant

Primary human lesional T cells were isolated from freshly taken skin biopsies of LP (n=5) and LE (n=3) patients (patient characteristics see Table S 1) by emigration towards an IL-2 gradient followed by expansion with α-CD3/α-CD28 stimulation as described previously (J Invest Dermatol, (2018), Case Rep Pathol 2021, 8819560 (2021)). Supernatants of expanded lesional T cells were generated by 3-day stimulation with 0.75 µg/mL α-CD3 (pre-coated, BD Biosciences) and 0.75 µg/mL soluble α-CD28 (BD Biosciences). A mixture of all 8 lesional supernatants at equimolar ratio was used for stimulation of 3D keratinocyte skin equivalents. Concentrations of key cytokines in the supernatant mix of lesional T cells were determined by a multiplex ELISA (Biorad) and listed in Table S4.

**Table S4. Composition of lesional LP/LE-TCS mix. Hu = human.**

| **Analyte** | **c (pg/ml)** |
|---|---|
| **Hu IL-1b (39)** | 200 |
| **Hu IL-1ra (25)** | 345 |
| **Hu IL-2 (38)** | 5.430 |
| **Hu IL-4 (52)** | 544 |
| **Hu IL-5 (33)** | 4.252 |
| **Hu IL-6 (19)** | 601 |
| **Hu IL-7 (74)** | 28 |
| **Hu IL-8 (54)** | 1.046 |
| **Hu IL-9 (77)** | 1.184 |
| **Hu IL-10 (56)** | 785 |
| **Hu IL-12(p70) (75)** | 86 |
| **Hu IL-13 (51)** | 20.371 |
| **Hu IL-15 (73)** | 114 |
| **Hu IL-17 (76)** | 382 |
| **Hu IL-22** | 3.290 |
| **Hu Eotaxin (43)** | 77 |
| **Hu FGF basic (44)** | 259 |
| **Hu G-CSF (57)** | 355 |
| **Hu GM-CSF (34)** | 34.800 |
| **Hu IFN-g (21)** | 9.678 |
| **Hu IP-10 (48)** | 2.580 |
| **Hu MCP-1(MCAF) (53)** | 575 |
| **Hu MIP-1a (55)** | 5.869 |
| **Hu PDGF-bb (47)** | 129 |
| **Hu MIP-1b (18)** | 11.120 |
| **Hu RANTES (37)** | 4.540 |
| **Hu TNF-a (36)** | 55.577 |
| **Hu VEGF (45)** | 514 |

### Tnf-α-driven systemic inflammatory response syndrome (SIRS) model

TNF-α driven systemic inflammatory response syndrome (SIRS) model was performed as described before (Immunity 35, 908-918 (2011*)*)*.* Briefly, male C57BL/6 mice (n=6 per group) were injected into the tail vain with a volume of 125 µl per 25 g mouse of either vehicle (15 % PEG 200; 8.4 % DMSO; 0.6 % NaCl) or a mixture of murine Tnf-α (0.3 mg/kg) and zVAD (6.7 mg/kg) prepared in vehicle. No Tnf-α/zVAD was administered to the vehicle group. 15 min after induction of hypothermia by application of Tnf-α/zVAD mix, animals were orally dosed with RIPK1 inhibitor or GSK`963. Body temperature was monitored every hour using radio-frequency identification (RFID) technology with IPTT-300 transponders that were injected subcutaneously 2-3 days before initiation of the experiment.

### Human lesional skin biopsy cultures

6 mm punch biopsies of lesional skin of LP patients (n=5) were obtained under local anaesthesia. Biopsies were divided into two parts and treated with the RIPK1 inhibitor (1 µM, SANOFI) or without as untreated control sample for 4 h in T cell media (RPMI 1640 medium with 1 % human serum, 0.1 mM non-essential aminoacids, 2 mM L-Glutamine, 1 mM sodium pyruvate and 100 U/mL penicillin/streptomycin). After stimulation, biopsies were stored in RNALater^{®} solution at -80 °C until RNA preparation.

### Cell Viability Assay

Cell viability of cultured keratinocytes was determined using CellTiter 96^{®} Aqueous NonRadioactive Cell Proliferation Assay (Promega, G5421) according to manufacturer's instructions. MTS/PMS reagent was used 1:6 diluted in keratinocyte medium without hydrocortisone and incubated for 1 h. Afterwards, supernatants were analysed by ELISA at 490 nm minus 700 nm as reference. To calculate the percentage of cell viability to ZS extinction values were normalized to the control stimulation zVAD + SMAC (100 %).

### Multiplex ELISA

Cell free supernatants derived from keratinocyte stimulation experiments or lesional TCS from LP/LE patients were analysed for 27 cytokines, chemokines and growth factors using the Bio-Plex Pro Human Cytokine 27-plex Assay (Bio-Rad Laboratories, Hercules, Calif) according to the manufacturer's recommendation.

### Histology of skin samples and 3D keratinocyte skin equivalents

Fixated skin tissue or 3D keratinocyte skin equivalents were embedded in paraffin. 5 µm sections were cut and dewaxed at 65 °C for 20 min. After rehydration, sections were stained with hematoxylin and eosin using standard methods. High resolution images (20x) were obtained with Evos microscope.

### Immunofluorescence staining

4 µm sections of paraffin-embedded skin biopsy samples were air-dried overnight at 37 °C, deparaffinized, rehydrated and heat treated at 95 °C for 32 min in Cell Conditioning Solution (Roche Diagnostics, #05279801001). Then, slides were incubated manually overnight at 4 °C with primary antibody, including monoclonal rabbit anti-human RIPK1 (Cell Signaling, CST3493, diluted 1:400), monoclonal rabbit anti-human RIPK1-pS166 (Cell Signaling, CST44590, diluted 1:200), monoclonal mouse anti-human RIPK3 (R&D Systems, MAB7604, diluted 1:500), monoclonal rabbit anti-human RIPK3-pS227 (Cell Signaling, CST93654, diluted 1:50), monoclonal rabbit anti-human MLKL (abcam, ab 184718, diluted 1: 100) and monoclonal rabbit anti-human MLKL-pS358 (abcam, ab187091, diluted 1:25). After washing with PBS (pH 7.4, GIBCO, #10010-056), sections were incubated with secondary antibody (diluted 1:300), including goat anti-rabbit AF750 (Invitrogen, #A21039) and goat anti-mouse IgG-AF647 (Invitrogen, #A21240) for 1 h at RT. Nuclei were counterstained with DAPI (1:3,000, Thermo Prod, #62247) 10 min at RT. Slides were mounted in Prolong^{™} Gold antifade reagent (Invitrogen, #P36930) and examined using a microscope slide scanner (Axio Scan.Z1 Zeiss, Germany) at 20x magnification.

### RNA preparation, cDNA synthesis and qPCR

RNA was isolated using QIAzol Lysis Reagent (Qiagen) and miRNeasy Mini Kit (Qiagen) according to manufacturer's protocol. mRNA was transcribed into cDNA with Applied Biosytems High Capacity cDNA Reverse Transcription Kit (Thermo Fisher Scientific). Gene expression was measured on an Applied Biosystems ViiA7 Real-Time PCR system (Thermo Fisher Scientific) using Fast Start Universal SYBRGreen Master Rox (Roche). Primers were ordered from Metabion (http://www.metabion.com) and are listed in supplementary Table S5. Data are shown as relative gene expression to untreated sample using 2^{-(ΔΔcT)} method.

**Table S5. Primer sequences. Fw = forward. rv = reverse.**

| **Gene** | **Sequence (5'-3')** | |
|---|---|---|
| ***18S*** | fw | GTAACCCGTTGAACCCCATT |
| | rv | CCATCCAATCGGTAGTAGCG |
| ***IFNa*** | fw | CTTGACTTGCAGCTGAGCAC |
| | rv | CAGAGTCACCCATCTCAGCA |
| ***IFNg*** | fw | GAGTGTGGAGACCATCAAGGA |
| | rv | TGGACATTCAAGTCAGTTACCGAA |
| ***TNF*** | fw | TGTTGTAGCAAACCCTCAAGC |
| | rv | ATGAGGTACAGGCCCTCTGA |
| ***IL1b*** | fw | AAGCCCTTGCTGTAGTGGTG |
| | rv | GAAGCTGATGGCCCTAAACA |
| ***IL1RA*** | fw | AATGCTGACTCAAAGGAGACGA |
| | rv | TTCTGAAGGCTTGCATCTTGC |
| ***CXCL9*** | fw | GAGTGCAAGGAACCCCAGTAG |
| | rv | GGTGGATAGTCCCTTGGTTGG |
| ***CXCL10*** | fw | AACCTCCAGTCTCAGCACCA |
| | rv | GAGAGAGGTACTCCTTGAATGCC |
| ***CXCL11*** | fw | TTGTTCAAGGCTTCCCCATGTT |
| | rv | CACTTTCACTGCTTTTACCCCA |
| ***CXCL5*** | fw | CAGACCACGCAAGGAGTTCA |
| | rv | TCTTCAGGGAGGCTACCACT |
| ***CXCL8*** | fw | ACCGGAAGGAACCATCTCAC |
| | rv | GGCAAAACTGCACCTTCACAC |
| ***CCL3*** | fw | GCTCTCTGCAACCAGTTCTCT |
| | rv | TCGCTTGGTTAGGAAGATGACA |
| ***CCL5*** | fw | GGTACCATGAAGGTCTCCGC |
| | rv | GGTGTCCGAGGAATATGGGG |
| ***CCL20*** | fw | TTTGCTCCTGGCTGCTTTGA |
| | rv | AGCAGTCAAAGTTGCTTGCTTC |

### Western blot

Keratinocytes were lysed in RIPA lysis buffer (Santa Cruz) supplemented with 1 mM sodium orthovanadate, 2mM PMSF and proteinase inhibitor cocktail (1:70) according to the manufacturer's instructions. Equal protein concentrations - determined by BCA protein assay - were resolved by SDS-PAGE using Bolt 4-12 % Bis-TrisPlus Gels and analysed by western blot using enhanced chemiluminescence. For staining following antibodies were used: rbαhpRIPK1(Ser166) (Cell signaling, 65746, 1:1,000 in BSA), mαhβ-ACTIN (SIGMA, A2228, 1:10,000 in milk) and αmHRP (Jackson, 115-035-166, 1:10,000 in milk).

### Statistical analysis

Data were analysed using GraphPad Prism 6 software and visualized as mean ± standard error of mean (SEM). Comparison of disease or treatment groups was performed as indicated in figure legends. Significance level was defined as p<0.05 (*), p<0.01 (**), p<0.001 (***) and p<0.0001 (****)

### RESULTS

### RIPK1 is specifically elevated in LP and LE and correlates to the strength of ID besides RIPK3 and MLKL

LP and LE typically have an ID, a subepidermal infiltrate leading to cell death of basal keratinocytes (Fig. 1A). To determine that necroptosis is a central pathogenic event in ID, the expression of *RIPK1, RIPK3* and *MLKL* in LP/LE as well as atopic dermatitis (AD) and psoriasis skin biopsies were analyzed and compared to intraindividual non-lesional skin. While gene expression of *MLKL* and *RIPK3* was significantly upregulated in lesional skin of all three diseases (*MLKL*: p<0.0001 for all; *RIPK3*: p<0.0001 for LP/LE, p=0.0140 for AD, p=0.0035 for Pso), *RIPK1* was exclusively elevated in LP/LE (p=0.0500) and downregulated in AD (p=0.0280) and psoriasis (p=0.0005) (Fig. 1B). Thus, while *RIPK3* and *MLKL* are upregulated in all three conditions, only *RIPK1,* an upstream mediator of necroptosis, is significantly elevated in LP/LE. Moreover, total gene counts of *RIPK1, RIPK3* and *MLKL* are significantly higher in LP/LE compared to AD and psoriasis (p<0.0001 for all). In addition, immunofluorescence confirmed enhanced pRIPK1, pRIPK3 and pMI,KL expression in the epidermis of LP lesions, which confirms the necroptosis pathway activation in LP (Fig. S1).

Next, the correlation of *RIPK1, RIPK3* and *MLKL* gene counts were tested to the grade of ID. For this, ID was rated by an expert dermatopathologist on a scale from 0 (no ID), 1 (mild ID), 2 (moderate ID) and 3 (severe ID) taking into account the strength of the inflammatory infiltrate and the extent of epidermal cell death. Here, a strong correlation were found between the grade of ID and gene counts of *RIPK1* (r=0.98; p=0.0157), *RIPK3* (r=0.98; p=0.0163) and *MLKL* (r=0.99; p=0.0071) (Fig. 1C). Thus, markers of necroptosis are highly expressed in type 1 mediated ISD and correlate to the ID. Of note, only *RIPK1* was specifically elevated in LP and LE, thus representing a potential upstream target for blocking necroptosis in these diseases.

### RIPK1 blockade prevents keratinocyte cell death and release of inflammatory cytokines

Based on these findings the effects of a RIPK1 inhibition by SAR443122, a novel, specific type III kinase inhibitor of RIPK1 were investigated on necroptosis *in vitro.* Primary human keratinocytes were stimulated with IFN-γ and TNF-α. As IFN-γ and TNF-α induce both apoptosis and necroptosis, zVAD (z-Val-Ala-DL-Asp(Ome)-fluoromethylketone) was added to block caspase dependent apoptosis and SMAC to achieve full activation of RIPK1 by preventing its ubiquitination. The RIPK1 inhibitor was demonstrated efficiently blocked RIPK1 autophosphorylation (Fig. S2). Stimulation of primary keratinocytes with IFN-γ, TNF-α, zVAD and SMAC led to 60 % cell death (43,6 % ± 7.6 % viability), an effect which was fully blocked by the RIPK1 inhibitor at a concentration of 4 nM (95.1 % ± 0.8 % viability) or higher (Fig. 2A). Furthermore, RIPK1 inhibition significantly diminished the release of IL-1β to basal levels in a dose dependent manner (p=0.0050 for 100 nM) (Fig. 2B). Interestingly, RIPK1 inhibition also resulted in significant reduction of IL-1α, IL-1RA, CXCL5, IL-8 (CXCL8), GM-CSF and CCL20 (Fig. 2C). Hence, these data show that necroptosis is a pathogenic event in LP and LE, as inhibition leads to downregulation of various immune pathways including IFN-γ and TNF-α. Blocking RIPK1 does not only affect necroptotic cell death, but also results in markedly decreased amount of pro-inflammatory cytokines and chemokines released by keratinocytes.

### RIPK1 inhibition normalizes skin architecture in three-dimensional skin equivalents

To test the effects of RIPK1 blockade in a preclinical model of LP and LE, we isolated T cells from LP (n=5) and LE (n=3) skin biopsies and generated T cell supernatants (TCS) by anti-CD3 and anti-CD28 stimulation. The mixed LP/LE-TCS contained TNF-α (55,577 pg/ml), GM-CSF (34,800 pg/ml), IL-13 (20,371 pg/ml) and IFN-γ (9,678 pg/ml) (Fig. 3A), thereby resembling the natural cytokine composition of keratinocytes in type 1 ISD. Next, three-dimensional skin equivalents were generated resembling the physiological layers of the human epidermis. When adding the LP/LE-TCS, cell swelling and cell death of keratinocytes were observed resulting in an enhanced epidermal thickness (102.0 ± 5.1 µM) compared to unstimulated controls (61.3 ± 5.4 µM) (Fig. 3B, 3C). In concordance with our previous observations, blocking RIPK1 prevented cell death, significantly reduced epidermal thickness (80.3 ± 5.1 µM, p<0.0001) and restored the normal epidermal architecture (Fig. 3B, 3C). In addition, RIPK1 inhibition reduced the release of II,-1RA (48.9 % ± 18.0 %, p=0.0175), TNF-α (70.3 % ± 6.3 %, p=0.0204), IL-8 (79.8 % ± 9.6 %, p=0.4757) and CCL20 (29.1 % ± 4.4 %, p<0.0001) compared to untreated controls (Fig. 3D).

### RIPK1 inhibitor blocks Tnf-α-induced hypothermia in vivo

Next, the effects of RIPK1 inhibition were assessed *in vivo* using a murine Tnf-α-driven systemic inflammatory response syndrome model (Immunity 35, 908-918 (2011*)*)*.* C57BL/6 mice (n=6 per group) were injected of either vehicle (15 % PEG 200; 8.4 % DMSO; 0.6 % NaCL 0.9 %) or a mixture of murine Tnf-α (0.3 mg/kg) and zVAD (16.7 mg/kg) into the tail vein. 15 minutes after induction of hypothermia, animals were orally dosed with the RIPK1 inhibitors SAR443122 or GSK`963, a previously described RIPK1 inhibitor ( Cell Death Discov 1, 15009 (2015*)*)*.* Body temperature was monitored up to 5 hours using radio-frequency identification technology with IPTT-300 transponders that were injected subcutaneously 2-3 days before initiation of the experiment (Fig. 4A). Animals receiving the Tnf-α/zVAD mixture rapidly responded with a significant decrease of body temperature within 5 hours from 37.3 ± 0.2 °C to 27.9 ± 0.2 °C and a delta temperature of -9.3 ± 0.3 °C (p=0.0002) compared to vehicle (-1.9 ± 0.5 °C). Oral administration of RIPK1 inhibitor led to a dose dependent increase of delta body temperature (1 mg/kg: -8.8 ± 0.5 °C; 5 mg/kg: -7.6 ± 1.2 °C; 15 mg/kg: -3.4 ± 1.3 °C, p=0.0007 to Tnf-α/zVAD) (Fig. 4B). Thus, RIPK1 inhibition efficiently inhibits Tnf-α induced necroptosis *in vivo.*

### RIPK1 inhibition downregulates target genes of LP in human skin biopsies ex vivo

Finally, to directly investigate the effects of RIPK1 inhibition in human tissue samples, *ex vivo* cultured human LP skin biopsies (n=5) were investigated. For this, LP skin biopsies were divided into two parts: One cultured with the RIPK1 inhibitor and one in pure medium for four hours (Fig. 5A). Of note, neither additional cytokines nor zVAD or SMAC were supplemented in these experiments. Genes specifically upregulated in LP compared to intraindividual non lesional skin are *IFNg, TNF, CXCL9, CXCL10, CXCL11, CXCL8* and *CCL3* (Fig. 5B). After RIPK1 inhibition a significant downregulation of *IFNg* (60 %, p=0.0076), *TNF* (60 %, p=0.0037), *CXCL9*/*10*/*11* (57/40/43 %, p=0.0128/0.0024/0.0045), *CCL3* (62 %, p=0.0174) and *IL1RA* (65 %, p=0.0123) were observed in LP skin biopsies compared to the autologous non-treated controls (Fig. 5C). Thus, RIPK1 inhibition not only affects necroptotic cell death, but normalizes several inflammatory pathways including Th1 related cytokines (*IFNg, TNF*) and IFN-γ responsive genes (*CXCL9*, *CXCL10, CXCL11*).

The disclosure is further characterized by the following embodiments.
Embodiment 1. A method of treating lichen planus in a subject in need thereof, comprising administering to the subject in need thereof a therapeutically effective amount of a RIPK1 inhibitor (RIPK1i).
Embodiment 2. The method according to embodiment 1, wherein RIPK1 inhibitor is (S)-5-benzyl- N-(5-methyl-4-oxo-2,3,4,5-tetrahydropyrido[3,2-b][1,4]oxazepin-3-yl)-4H-1,2,4-triazole-3-carboxamide, and/or a pharmaceutically acceptable salt, tautomer, stereoisomer or mixture of stereoisomers thereof.
Embodiment 3. The method according to embodiment 1 or embodiment 2, wherein the therapeutically effective amount of the RIPK1i is sufficient to reduce the level of one or more of IFNg, TNF, CXCL9/10/11, CCL3 and IL1RA.
Embodiment 4. A method of treating cutaneous lupus erythematosus in a subject in need thereof, comprising administering to the subject in need thereof a therapeutically effective amount of a RIPK1 inhibitor (RIPK1i), wherein the therapeutically effective amount of the RIPK1i is sufficient to reduce the level of one or more of IFNg, TNF, CXCL9/10/11, CCL3 and IL1RA.
Embodiment 5. The method according to embodiment 4, wherein RIPK1 inhibitor is (S)-5-benzyl- N-(5-methyl-4-oxo-2,3,4,5-tetrahydropyrido[3,2-b][1,4]oxazepin-3-yl)-4H-1,2,4-triazole-3-carboxamide, and/or a pharmaceutically acceptable salt, tautomer, stereoisomer or mixture of stereoisomers thereof.
Embodiment 6. A method of normalizing or improving skin architecture in a subject in need thereof, comprising administering to the subject in need thereof a therapeutically effective amount of a RIPK1i.
Embodiment 7. The method according to embodiment 6, wherein RIPK1 inhibitor is (S)-5-benzyl- N-(5-methyl-4-oxo-2,3,4,5-tetrahydropyrido[3,2-b][1,4]oxazepin-3-yl)-4H-1,2,4-triazole-3-carboxamide, and/or a pharmaceutically acceptable salt, tautomer, stereoisomer or mixture of stereoisomers thereof.
Embodiment 8. A method of normalizing or restoring normal epidermal in a subject in need thereof, comprising administering to the subject in need thereof a therapeutically effective amount of a RIPK1i.
Embodiment 9. The method according to embodiment 8, wherein RIPK1 inhibitor is (S)-5-benzyl- N-(5-methyl-4-oxo-2,3,4,5-tetrahydropyrido[3,2-b][1,4]oxazepin-3-yl)-4H-1,2,4-triazole-3-carboxamide, and/or a pharmaceutically acceptable salt, tautomer, stereoisomer or mixture of stereoisomers thereof. Embodiment 10. A method of reducing epidermal thickness in a subject in need thereof, comprising administering to the subject in need thereof a therapeutically effective amount of a RIPK1i.
Embodiment 11. The method according to embodiment 10, wherein RIPK1 inhibitor is (S)-5-benzyl- N-(5-methyl-4-oxo-2,3,4,5-tetrahydropyrido[3,2-b][1,4]oxazepin-3-yl)-4H-1,2,4-triazole-3-carboxamide, and/or a pharmaceutically acceptable salt, tautomer, stereoisomer or mixture of stereoisomers thereof.

## Claims

1. A RIPK1 inhibitor (RIPK1i) for use in treating lichen planus in a subject in need thereof, wherein said RIPK1 inhibitor is for administration to the subject in need thereof in a therapeutically effective amount.

2. The RIPK1 inhibitor for use according to claim 1, wherein RIPK1 inhibitor is (S)-5-benzyl-N-(5-methyl-4-oxo-2,3,4,5-tetrahydropyrido[3,2-b][1,4]oxazepin-3-yl)-4H-1,2,4-triazole-3-carboxamide, and/or a pharmaceutically acceptable salt, tautomer, stereoisomer or mixture of stereoisomers thereof.

3. The RIPK1 inhibitor for use according to claim 1 or claim 2, wherein the therapeutically effective amount of the RIPK1i is sufficient to reduce the level of one or more of IFNg, TNF, CXCL9/10/11, CCL3 and IL1RA.

4. A RIPK1 inhibitor (RIPK1i) for use in treating cutaneous lupus erythematosus in a subject in need thereof, wherein said RIPK1 inhibitor is for administration to the subject in need thereof in a therapeutically effective amount, wherein the therapeutically effective amount of the RIPK1i is sufficient to reduce the level of one or more of IFNg, TNF, CXCL9/10/11, CCL3 and IL1RA.

5. The RIPK1 inhibitor for use according to claim 4, wherein RIPK1 inhibitor is (S)-5-benzyl-N-(5-methyl-4-oxo-2,3,4,5-tetrahydropyrido[3,2-b][1,4]oxazepin-3-yl)-4H-1,2,4-triazole-3-carboxamide, and/or a pharmaceutically acceptable salt, tautomer, stereoisomer or mixture of stereoisomers thereof.

6. A RIPK1 inhibitor (RIPK1i) for use in normalizing or improving skin architecture in a subject in need thereof, wherein said RIPK1 inhibitor is for administration to the subject in need thereof in a therapeutically effective amount.

7. The RIPK1 inhibitor for use according to claim 6, wherein RIPK1 inhibitor is (S)-5-benzyl-N-(5-methyl-4-oxo-2,3,4,5-tetrahydropyrido[3,2-b][1,4]oxazepin-3-yl)-4H-1,2,4-triazole-3-carboxamide, and/or a pharmaceutically acceptable salt, tautomer, stereoisomer or mixture of stereoisomers thereof.

8. A RIPK1 inhibitor (RIPK1i) for use in normalizing or restoring normal epidermal in a subject in need thereof, wherein said RIPK1 inhibitor is for administration to the subject in need thereof in a therapeutically effective amount.

9. The RIPK1 inhibitor for use according to claim 8, wherein RIPK1 inhibitor is (S)-5-benzyl-N-(5-methyl-4-oxo-2,3,4,5-tetrahydropyrido[3,2-b][1,4]oxazepin-3-yl)-4H-1,2,4-triazole-3-carboxamide, and/or a pharmaceutically acceptable salt, tautomer, stereoisomer or mixture of stereoisomers thereof.

10. A RIPK1 inhibitor (RIPK1i) for use in reducing epidermal thickness in a subject in need thereof, wherein said RIPK1 inhibitor is for administration to the subject in need thereof in a therapeutically effective amount.

11. The RIPK1 inhibitor for use according to claim 10, wherein RIPK1 inhibitor is (S)-5-benzyl-N-(5-methyl-4-oxo-2,3,4,5-tetrahydropyrido[3,2-b][1,4]oxazepin-3-yl)-4H-1,2,4-triazole-3-carboxamide, and/or a pharmaceutically acceptable salt, tautomer, stereoisomer or mixture of stereoisomers thereof.
